# EUROPÄISCHE PATENTSCHRIFT

(11) **EP 0 906 389 B1**
(45) Veröffentlichungstag und Bekanntmachung des Hinweises auf die Patenterteilung: **04.10.2000**
(21) Anmeldenummer: 97927026.1
(22) Anmeldetag: 22.05.1997
(51) Int. Cl.: C11D 1/74, C11D 3/20, C11D 17/00, C07C 69/675, C07C 69/70, C07C 69/704

(54) **VERWENDUNG VON HYDROXYCARBONSÄUREESTERN ALS VERDICKUNGSMITTEL**
USE OF HYDROXYCARBOXYLIC ACID ESTERS AS THICKENERS
UTILISATION D'ESTERS D'ACIDE HYDROXYCARBOXYLIQUE COMME EPAISSISSANTS

(30) Priorität: 03.06.1996 DE 19622214
(43) Veröffentlichungstag der Anmeldung: 07.04.1999
(73) Patentinhaber: Cognis Deutschland GmbH, 40589 Düsseldorf (DE)
(72) Erfinder: PI SUBIRANA, Rafael, E-08400 Granollers (ES); PRAT QUERALT, Ester, E-08238 Calella (ES); BIGORRA LLOSAS, Joaquim, E-08203 Sabadell (ES)
(86) Internationale Anmeldenummer: EP9702619
(87) Internationale Veröffentlichungsnummer: WO9746653

(56) Entgegenhaltungen:
- EP-A- 0 199 131
- US-A- 5 302 377

## Beschreibung

Die Erfindung betrifft die Verwendung von Hydroxycarbonsäureestern, die man erhält, indem man ausgewählte Hydroxycarbonsäuren in an sich bekannter Weise mit ausgewählten Hydroxylverbindungen umsetzt, als Verdickungsmittel für die Herstellung oberflächenaktiver Mittel.

### Stand der Technik

Oberflächenaktive Mittel, wie beispielsweise Handgeschirrspülmittel oder Haarshampoos, Flüssigwaschmittel oder Duschgele, stellen mehr oder weniger konzentrierte wäßrige Tensidzubereitungen dar, denen allen gemeinsam ist, daß sie eine Viskosität aufweisen müssen, die einerseits niedrig genug ist, um eine problemlose Handhabung durch den Verbraucher sicherzustellen, andererseits aber auch hoch genug ist, um eine sparsame Dosierung zu ermöglichen. Bei Präparaten, die bereits in einer Anwendungskonzentration in den Handel gelangen und vom Verbraucher nicht noch einmal vor der eigentlichen Anwendung verdünnt werden müssen, bedeutet dies, daß die wasserdünnen Tensidlösungen auf eine höhere Viskosität eingestellt werden müssen. In vielen Fällen gelingt dies durch die Zugabe von Elektrolytsalzen oder Polymeren, in kritischen Fällen, zu denen beispielsweise anionische Tenside mit innenständigen polaren Gruppen und insbesondere auch Zuckertenside vom Typ der Alkylglucoside zählen, schlägt diese Maßnahme indes fehl. So kann durch Zugabe von Kochsalz beispielsweise die Viskosität von Alkylglucosidlösungen deutlich herabgesetzt werden.

Aus dem Stand der Technik sind eine Reihe von Verdickungsmitteln bekannt, mit deren Hilfe man auch die Viskosität der vorgenannten "problematischen" Tenside mehr oder minder regulieren kann. Ein geeignetes Mittel stellen beispielsweise die aus der Deutschen Patentanmeldung **DE-A1 3817415** (Henkel) bekannten Fettalkoholpolyglycolether mit eingeengter Homologenverteilung dar. Andere geeignete Verdickungsmittel, nämlich hochethoxylierte Glycerinester, werden in der Deutschen Patentanmeldung **DE-A1 4137317**- (Henkel) und der Französischen Patentanmeldung **FR-A 2534923** (Th.Goldschmidt) vorgeschlagen. Ester der Citronensäure mit Fettalkoholen sind beispielsweise aus der Französischen Patentanmeldung **FR-A 2623422** (L'Oréal) bekannt. In der Praxis zeigt sich jedoch, daß diese Mittel eine nicht ausreichende bzw. nicht ausreichend stabile Viskositätssteigerung hervorrufen, so daß nach wie vor ein Bedürfnis nach verbesserten Verdickungsmittel für die Herstellung von oberflächenaktiven Zubereitungen besteht.

Die Aufgabe der Erfindung hat somit darin bestanden, Verdickungsmittel zur Verfügung zu stellen, die es erlauben, auch wäßrige Lösungen "problematischer" Tenside zuverlässig und dauerhaft zu verdikken, ohne dabei die anwendungstechnischen Eigenschaften der Präparate nachteilig zu beeinflussen. Gleichzeitig sollten die Produkte eine ausgezeichnete ökotoxikologische Verträglichkeit aufweisen.

### Beschreibung der Erfindung

Gegenstand der Erfindung ist die Verwendung von Hydroxycarbonsäureestern, die man erhält, indem man Hydroxycarbonsäuren ausgewählt aus der Gruppe, die gebildet wird von Weinsäure, Äpfelsäure und Citronensäure, mit Fettalkoholpolyglycolethern der Formel (**I**),

**R**^{**1**}**O(CH**_{**2**}**CH**_{**2**}**O)**_{**n**}**H** **(I)**

in der R¹ für einen Alkyl- und/oder Alkenylrest mit 6 bis 22 Kohlenstoffatomen und n für Zahlen von 20 bis 150 steht, in an sich bekannter Weise umsetzt, als Verdickungsmittel für die Herstellung oberflächenaktiver Mittel.

Überraschenderweise wurde gefunden, daß die neuen Ester bezüglich wäßriger Tensidlösungen eine stark verdickende Wirkung zeigen, so daß sich auch in schwerverdickbaren Systemen, wie beispielsweise Zuckertensiden vom Typ der Alkylglucoside oder Fettsäure-N-methylglucamide, eine ausreichend hohe und stabile Viskosität einstellen läßt. Ein weiterer Vorteil dieser Gruppe von Verdickungsmitteln besteht darin, daß sie eine hohe ökotoxikologische Verträglichkeit aufweisen und sich problemlos sowohl in der Hitze als auch in der Kälte in kosmetische oder pharmazeutische Zubereitungen einarbeiten lassen.

### Hydroxycarbonsäuren

Als geeignete Hydroxycarbonsäuren kommen Weinsäure, Äpfelsäure und insbesondere Citronensäure in Betracht, die wasserfrei, vorzugsweise aber kristallwasserhaltig eingesetzt werden können.

### Fettalkoholpolyglycolether

Fettalkoholpolyglycolether, die im Sinne der vorliegenden Erfindung als Ausgangsstoffe in Betracht kommen, stellen handelsübliche Anlagerungsprodukte von durchschnittlich 20 bis 150, vorzugsweise 30 bis 120 und insbesondere 40 bis 100 Mol Ethylenoxid an technische Fettalkohole mit 6 bis 22, vorzugsweise 12 bis 18 und insbesondere 16 bis 18 Kohlenstoffatomen dar. Typische Beispiele sind die entsprechenden Ethoxylate von Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Vorzugsweise werden Anlagerungsprodukte von 40 bis 100 Mol Ethylenoxid an Cetearylalkohol, Talgfettalkohol oder Palmalkohol eingesetzt.

In Summe bevorzugt sind Hydroxycarbonsäureester, die sich von der Citronensäure und Fettalkoholpolyglycolethern der Formel (I) ableiten, in der R¹ für einen Alkylrest mit 16 bis 18 Kohlenstoffatomen und n für Zahlen von 40 bis 100 steht.

### Veresterung

Die Umsetzung der Hydroxycarbonsäuren mit den Fettalkoholpolyglycolethem kann in an sich bekannter Weise durchgeführt werden. Es empfiehlt sich, die Reaktion in Gegenwart eines sauren Katalysator, wie beispielsweise Methansulfonsäure oder p-Toluolsulfonsäure durchzuführen, die man in Mengen von 0,1 bis 1 und vorzugsweise 0,2 bis 0,7 Gew.-% - bezogen auf die Einsatzstoffe - verwenden kann. Zur Verbesserung der Farbqualität der Reaktionsprodukte hat es sich weiterhin als vorteilhaft erwiesen, ein Reduktionsmittel wie beispielsweise unterphosphorige Säure oder Natriumhypophosphit mitzuverwenden, wobei die Einsatzmenge bei etwa 1 bis 50 Gew.-% bezogen auf den Katalysator liegen sollte. Die Hydroxycarbonsäuren und die Polyglycolether werden in der Regel in Mengen einsetzt, die einem molaren Verhältnis von Carboxyl- zu Hydroxylgruppen von 1 : 1 bis 3 : 1 und vorzugsweise 2 : 1 bis 2,5 : 1 entsprechen. Die Veresterung erfolgt in der Wärme bei Temperaturen im Bereich von 100 bis 200°C, vorzugsweise unter vermindertem Druck. Im Falle wasserlöslicher Produkte kann der Fortgang der Reaktion kann über die Parameter Säurezahl und Viskosität verfolgt werden. Üblicherweise führt man dann die Veresterung solange fort, bis die Säurezahl unter einen Wert von 20, vorzugsweise unter 10 abgesunken ist. Hierbei gilt es jedoch zu beachten, daß Ester gleicher Säurezahl sehr wohl unterschiedliche verdickende Wirkungen aufweisen können. Ester mit besonders vorteilhaften Eigenschaften werden dann erhalten, wenn sie nicht nur das Kriterium der niedrigen Säurezahl erfüllen, sondern eine 5 Gew.-%ige Probe des Esters in Wasser eine Viskosität nach Brookfield von mindestens 2.000 mPas, vorzugsweise mindestens 4.000 mPas und insbesondere mindestens 7.000 mPas aufweist. Zur Herstellung dieser bevorzugten Ester erhitzt man die Reaktionsprodukte solange weiter, bis eine Probe die gewünschte Viskosität zeigt.

Die Ester verfügen über stark verdickende Eigenschaften; sie dienen daher als Verdickungsmittel zur Herstellung von wäßrigen oberflächenaktiven Mitteln. Typische Beispiel hierfür sind Flüssigwaschmittel, Handgeschirrspülmittel, Wäscheweichspülmittel und insbesondere kosmetische bzw. pharmazeutische Zubereitungen wie etwa Haarshampoos, Duschgele, Schaumbäder, Haarconditioner, Hautlotionen, Cremes, Salben und dergleichen.

### Tenside

Die oben genannten Mittel können als weitere Bestandteile anionische, nichtionische, kationische und/oder amphotere bzw. zwitterionische Tenside enthalten. Typische Beispiele für **anionische Tenside** sind Alkylbenzolsulfonate, Alkansulfonate, Olefinsulfonate, Alkylethersulfonate, Glycerinethersulfonate, a-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligoglykoside, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen und Esterquats, insbesondere quaternierte Fettsäuretrialkanolaminester-Salze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöladditive", Thieme Verlag, Stuttgart, 1978, S. 123-217** verwiesen. Vorzugsweise werden die neuen Ester zur Verdickung von wäßrigen Tensidlösungen eingesetzt, die Alkyl- und/oder Alkenyloligoglykoside, Fettsäure-N-alkylglucamide, Fettalkoholethersulfate, Sulfosuccinate, Betaine und/oder Esterquats enthalten.

### Hilfs- und Zusatzstoffe

Werden die neuen Ester als Verdickungsmittel für kosmetische bzw. pharmazeutische Zubereitungen, wie beispielsweise Haar- oder Hautbehandlungsmittel, eingesetzt, können diese als weitere Hilfs- und Zusatzstoffe Ölkörper, Emulgatoren, Überfettungsmittel, Stabilisatoren, Wachse, Konsistenzgeber, Co-Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzfilter, Insektenrepellentien, Selbstbräuner, Farb- und Duftstoffe enthalten.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, Ester von C₆-C₂₂-Fett-alkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate Guerbetcarbonate, Dialkylether, Siliconöle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxid-anlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Lauryl-glucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472 und DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl* Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als Konsistenzgeber kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Co-Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentrimamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als Perlglanzwachse können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Zur Verbesserung des Fließverhaltens können ferner **Hydrotrope** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als Selbstbräuner eignet sich Dihydroxyaceton. Als **Farbstoffe** können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel, die durch Beispiele 1-4 illustriert wird, kann durch übliche Kalt- oder Heißprozesse erfolgen; vorzugsweise arbeitet man nach der Phaseninversionstemperatur-Methode.

### Beispiele

**Beispiel 1 : Verhältnis -COOH/-OH = 1,5 : 1**. In einer 1,5-I-Rührapparatur wurden 906 g (0,19 mol) eines Talgfettalkohol+100 EO-Adduktes (Hydroxylzahl 24,15 mg KOH/g) vorgelegt und auf 90°C vorgewärmt. Anschließend wurden 24 g (0,114 mol) Citronensäure-Monohydrat, 0,65 g unterphosphorige Säure und 4,2 g Methansulfonsäure zugegeben. Die Reaktionsmischung wurde auf 160°C erhitzt und dabei ein Vakuum von 40 mbar angelegt. Die Mischung wurde 2 h bei dieser Temperatur gerührt und der Druck dabei schrittweise bis auf 5 mbar abgesenkt. Der Verlauf der Reaktion wurde über die Säurezahl und die Viskosität des entstandenen Esters gemäß Tabelle 1 kontrolliert:

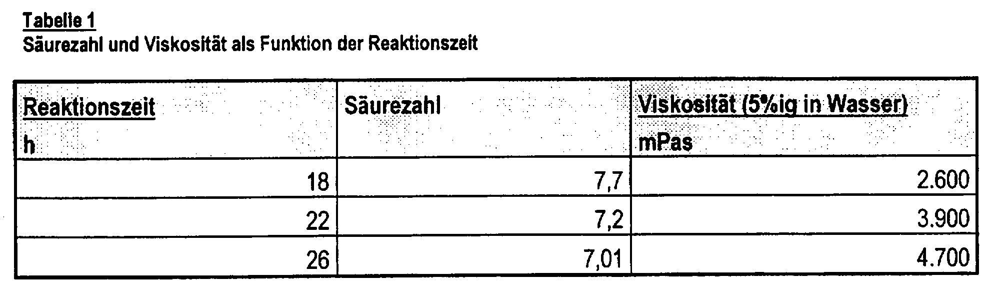

**Beispiel 2 :Verhältnis -COOH/-OH = 2:1.** Analog Beispiel 1 wurden 868 g (0,18 g) Cetylstearylalkohol+100EO-Addukt mit 31 g (0,148 mol) Citronensäure-Monohydrat, 0,65 g unterphosphorige Säure und 4,2 g Methansulfonsäure verestert. Der Verlauf der Reaktion wurde über die Säurezahl und die Viskosität des entstandenen Esters gemäß Tabelle 2 kontrolliert:

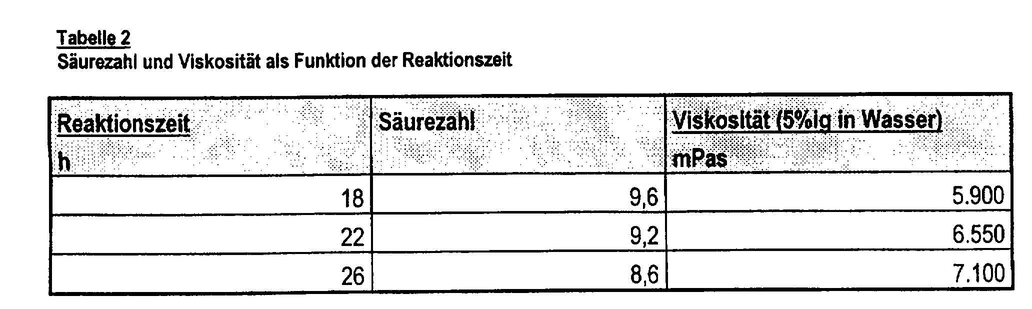

**Beispiel 3 : Verhältnis -COOH/-OH = 2,5 : 1.** Analog Beispiel 1 wurden 820 g (0,4 mol) Cetylstearylalkohol+40 EO-Addukt (Hydroxylzahl 29,1 mg KOH/g) mit 74,4 (0,354 mol) Citronensäure-Monohydrat, 0,59 g unterphosphoriger Säure und 3,8 g Methansulfonsäure verestert. Nach 25 h wurde ein Reaktionsprodukt erhalten, das eine Restsäurezahl von 17,5 aufwies.

**Beispiel 4 : Verhältnis -COOH/-OH = 2,0 : 1.** Analog Beispiel 3 wurden 800 g (0,4 mol) Cetearylalkohol+40 EO-Addukt mit 58,1 g (0,277 mol) Citronensäure-Monohydrat, 0,57 g unterphosphoriger Säure und 3,7 g Methansulfonsäure umgesetzt. Nach 25 h wurde ein Ester erhalten, der eine Restsäurezahl von 15,1 aufwies.

**Beispiele 5 und 6, Vergleichsbeispiel V1**. Die verdickende Wirkung der Citronensäureeester wurde bezüglich einer Tensidmischung aus Alkylglucosiden und Ethersulfaten untersucht. Die Bestimmung erfolgte nach der Brookfield-Methode in einen RVT-Viskosimeter (20°C, 10 Upm, Spindel 1). Die Verwendung in den Rezepturen R1 und R2 ist erfindungsgemäß, in der Vergleichsrezeptur R3 wurde als Verdickungsmittel PEG-150 Distearate getestet. Die Ergebnisse sind in Tabelle 3 zusammengefaßt. Man erkennt, daß die erfindungsgemäß eingesetzten Ester eine signifikant höhere Viskosität bewirken.

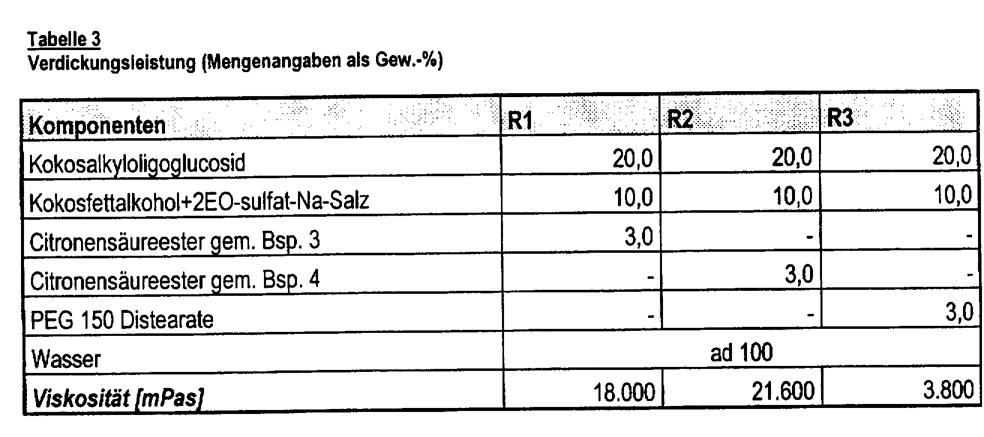

## Patentansprüche

1. Verwendung von Hydroxycarbonsäureestern, dadurch erhältlich, daß man Hydroxycarbonsäuren ausgewählt aus der Gruppe, die gebildet wird von Weinsäure, Äpfelsäure und Citronensäure, mit Fettalkoholpolyglycolethern der Formel (**I**) in an sich bekannter Weise umsetzt,
**R**^{**1**}**O(CH**_{**2**}**CH**_{**2**}**O)**_{**n**}**H (I)**
in der R¹ für einen Alkyl- und/oder Alkenylrest mit 6 bis 22 Kohlenstoffatomen und n für Zahlen von 20 bis 150 steht, als Verdickungsmittel für die Herstellung von oberflächenaktiven Mitteln.

2. Verwendung nach Anspruch 1, **dadurch gekennzeichnet,** daß man Ester der Citronensäure mit Fettalkoholpolyglycolethern der Formel (I) einsetzt, in der R¹ für einen Alkylrest mit 16 bis 18 Kohlenstoffatomen und n für Zahlen von 40 bis 100 steht.

3. Verwendung nach den Ansprüchen 1 und 2 , **dadurch gekennzeichnet,** daß man wäßrige Lösungen von Alkyl- und/ oder Alkenyloligoglykosiden verdickt.

## Claims

1. The use of hydroxycarboxylic acids obtainable by reacting hydroxycarboxylic acids selected from the group consisting of tartaric acid, malic acid and citric acid with fatty alcohol polyglycol ethers corresponding to formula (**I**):
**R**^{**1**}**O(CH**_{**2**}**CH**_{**2**}**O)**_{**n**}**H (I)**
in which R¹ is an alkyl and/or alkenyl group containing 6 to 22 carbon atoms and n is a number of 20 to 150,
by methods known per se as thickeners for the production of surface-active formulations.

2. The use claimed in claim 1, characterized in that esters of citric acid with fatty alcohol polyglycol ethers corresponding to formula (I), where R¹ is an alkyl group containing 16 to 18 carbon atoms and n is a number of 40 to 100, are used.

3. The use claimed in claims 1 and 2, characterized in that aqueous solutions of alkyl and/or alkenyl oligoglycosides are thickened.

## Revendications

1. Utilisation d'esters d'acide hydroxycarboxylique accessible en ce qu'
on fait réagir d'une manière connue en soi des acides hydroxycarboxyliques choisis dans le groupe formé de l'acide tartrique, de l'acide malique, et de l'acide citrique avec des éthers d'alcool gras et de polyglycol de formule (I),
R¹O(CH₂CH₂O)nH (I)
dans laquelle R¹ représente un radical alkyle et/ou alkényle ayant de 6 à 22 atomes de carbone et n des nombres allant de 20 à 150,
en tant qu'agent épaississant pour la préparation de produits actifs sur la tension superficielle.

2. Utilisation selon la revendication 1,
caractérisée en ce qu'
on met en oeuvre un ester d'acide citrique avec un éther d'alcool gras et de polyglycol de formule (I), dans laquelle R¹ représente un radical alkyle ayant de 16 à 18 atomes de carbone et n représente des nombres allant de 40 à 100.

3. Utilisation selon les revendications 1 et 2,
caractérisée en ce qu'
on épaissit des solutions aqueuses d'alkyl- et/ou d'alkényloligoglycosides.
